# FASCICULE DE BREVET EUROPEEN

(11) **EP 0 490 812 B1**
(45) Date de publication et mention de la délivrance du brevet: **17.01.1996**
(21) Numéro de dépôt: 91810931.5
(22) Date de dépôt: 28.11.1991
(51) Int. Cl.: A61B 17/60

(54) **Fixateur externe pour l'ostéosynthèse**
Äussere Fixatur zur Osteosynthese
External fixator for osteosynthesis

(30) Priorité: 10.12.1990 CH 3891/90
(43) Date de publication de la demande: 17.06.1992
(73) Titulaire: JAQUET ORTHOPEDIE S.A., CH-1228 Plan-les-Ouates (CH)
(72) Inventeur: Mata, Jacques, CH-1163 Etoy (CH); Wagenknecht, Marcel, CH-1219 Le Lignon (CH)
(74) Mandataire: Dietlin, Henri

(56) Documents cités:
- EP-A- 0 011 258
- EP-A- 0 216 563
- EP-A- 0 227 594
- DE-A- 2 718 515

## Description

La présente invention est du domaine de la santé et concerne plus particulièrement un fixateur externe d'os ou fragments osseux utilisé en traumatologie.

Depuis longtemps on a développé des fixateurs externes comportant des fiches insérées dans les fragments osseux qui sont reliées à un cadre ou à une ou plusieurs barres de liaison. Dans la plupart des cas, et pour assurer une stabilité maximale, on a l'habitude de disposer au moins deux fiches de part et d'autre de la fracture. Généralement, les fiches sont insérées en fonction de la position dans laquelle on prévoit d'installer le cadre du fixateur relativement au membre à soigner. Souvent elles sont disposées en utilisant un gabarit de perçage correspondant à l'étau de serrage des fiches prévu. Par conséquent les fiches sont parfois introduites dans des régions du corps que le praticien préférerait éviter, car trop proches de nerfs ou d'artères par exemple.

Pour pallier cet inconvénient, on a déjà proposé un fixateur externe utilisable quelles que soient les positions des fiches insérées préalablement dans les fragments osseux en traversant des régions préférentielles. Le brevet EP-A-0.216.563 décrit un fixateur qui comporte un joint universel permettant d'orienter les fiches par rapport aux barres de liaison avec un plus grand degré de liberté.

La présente invention propose un nouveau joint universel grâce auquel le praticien peut placer tout d'abord les fiches en fonction de la configuration de la fracture d'une part, et selon l'anatomie de chaque patient d'autre part. C'est alors seulement qu'il mettra en place les composants formant l'ensemble du fixateur externe.

Ces composants sont conçus pour être mis en place par rapport à des fiches déjà insérées dans des fragments osseux, et permettent de modifier à volonté le nombre des barres de liaison. Grâce au joint à rotule selon l'invention, on peut aisément remplacer la rotule selon la configuration finale désirée. En outre une seule opération de serrage permet d'immobiliser les barres de liaison par rapport aux fiches ou à d'autres barres.

Le fixateur selon l'invention comporte :
- des fiches insérées dans les fragments osseux,
- une ou plusieurs barres de positionnement constituant le cadre extérieur du fixateur,
- un ou plusieurs raccords d'articulation entre les barres et les fiches ou les barres,
les fiches étant au moins indirectement solidaires d'un joint universel à rotule amovible apte à assurer le positionnement indépendant des fiches par rapport à l'une desdites barres.

Il est caractérisé en ce que le joint universel est composé de :
- un corps partiellement sphérique constituant une rotule,
- une première partie munie d'une première creusure de forme générale sphérique,
- une seconde partie munie d'un passage longitudinal et d'un dégagement faisant face à la première creusure,
- un élément de blocage muni d'une seconde creusure de forme générale sphérique et disposé dans ledit dégagement,
- au moins une vis de serrage disposée sensiblement perpendiculairement audit passage longitudinal et apte à bloquer à la fois la rotule et la pièce cylindrique (barre ou fiche) introduite dans le passage longitudinal.

Grâce à ce système, le praticien met en place les fiches insérées dans des fragments osseux, en tenant compte uniquement de critères anatomiques, il maintient celles-ci dans un joint universel selon l'invention, soit directement soit avec un étau intermédiaire, et choisit le type de rotule à utiliser avant de mettre en place les barres de liaison et de réduire la fracture osseuse sous rayons X. Il peut en outre renforcer la rigidité du fixateur par des raccords et des barres supplémentaires que l'on peut partiellement retirer après le début de la consolidation osseuse.

Le dessin annexé représente, à titre d'exemples non limitatifs, des formes d'exécution de l'objet de la présente invention.

La figure 1 est une vue schématique d'un fixateur utilisé dans un montage sur un os long et comportant des joints universels selon l'invention.

La figure 2 est une vue, en perspective éclatée, d'un joint universel pour l'orientation et la fixation de deux pièces cylindriques.

La figure 3 est une vue en coupe longitudinale du joint de la figure 2.

La figure 4 est une coupe transversale selon IV-IV du joint de la figure 3.

La figure 5 est une vue en bout de la rotule utilisée dans le joint de la figure 2.

La figure 6 est une coupe longitudinale selon VI-VI à la figure 5.

La figure 7 est une vue en perspective d'un étau d'orientation et de serrage des fiches osseuses, utilisé conjointement avec le joint universel selon l'invention.

La figure 8 est une coupe longitudinale de l'étau de la figure 7.

La figure 9 est une vue en perspective d'une barre de liaison et d'un écrou de liaison à un autre composant.

La figure 10 est une coupe longitudinale de l'écrou de la figure 9.

La figure 11 est une vue en perspective d'une fiche osseuse, munie d'une douille de serrage.

La figure 12 représente les constituants de la douille de la figure 11, vus en coupe longitudinale dans la moitié supérieure et latéralement dans la moitié inférieure du dessin.

La figure 13 est une vue en perspective d'un raccord télescopique de deux pièces cylindriques, mises bout à bout.

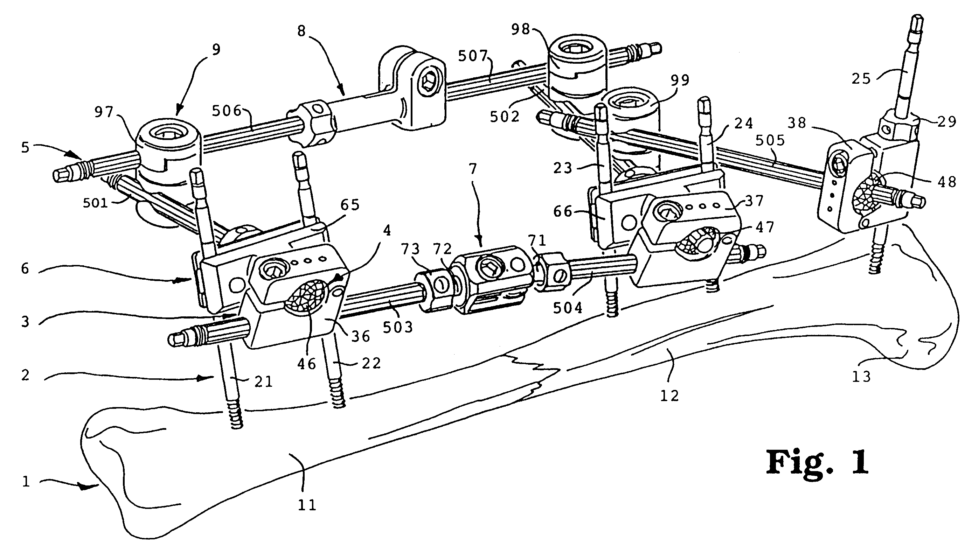

Dans la vue générale d'un fixateur de la figure 1 on a représenté un os 1 dans lequel sont insérées des fiches 2. Le fixateur comporte des joints universels 3 comportant des rotules 4 et destinés à l'orientation, au moins indirectement, des fiches par rapport aux barres 5 formant le cadre du fixateur. On peut en outre utiliser un étau 6 de serrage des fiches, ainsi que différents raccords disposés entre les barres, tels qu'une articulation 7, un raccord télescopique 8 et un raccord orientable 9.

On notera encore à la figure 1 que l'os 1 est fracturé en deux fragments 11 et 12, recevant respectivement les fiches 21, 22 et 23 à 25.

Les fiches 21 et 22 sont toutes deux serrées dans un étau 65 dont une mâchoire coopère avec une rotule 46 serrée dans le joint universel 36, et dont l'autre mâchoire est solidaire d'une barre de liaison 501 maintenue dans un raccord orientable de positionnement 97. De même, les fiches 23 et 24 sont fixées dans un étau 66 coopérant d'un côté avec la rotule 47 d'un joint universel 37 et de l'autre côté avec une barre de liaison 502 maintenue dans un raccord orientable 98. Il est à noter que la rotule 46 est disposée en bout d'une barre solidaire de l'étau 65 tandis que la rotule 47 présente un passage central pour la barre de liaison à l'étau 66, ce qui permet d'ajuster la distance entre le joint et l'étau. Comme on le verra plus en détail par la suite des passages longitudinaux, pratiqués dans les joints 36 et 37, reçoivent les barres de positionnement 503 et 504 qui sont orientables l'une par rapport à l'autre grâce au dispositif 7 d'articulation et de blocage. La fiche 25 est insérée dans l'épiphyse 13 et est directement fixée dans le joint universel 38 dont le corps sphérique 48 est apte à bloquer une barre 505 complétant le cadre du fixateur en venant s'ajouter à la barre 502 au moyen de raccord orientable 99.

En variante, on pourrait supprimer les étaux 65 et 66, et fixer directement chaque fiche 21 à 24 comme celle représentée sous référence 25 dans un joint universel qui serait monté le long des barres 503 ou 504.

Entre les raccords orientables 97 et 98, on a représenté une barre de rigidification de l'ensemble qui peut être supprimée après un début de consolidation de la fracture. A la figure 1, on a représenté une barre en deux parties 506 et 507 séparées par un raccord télescopique 8, afin de présenter ce dernier dans la vue générale, bien qu'il ne soit pas obligatoire dans cette configuration où la longueur de la barre peut être ajustée par rapport aux raccords orientables 97 et 98.

Pour assurer un blocage optimum des composants représentés, on utilisera de préférence des barres de section polygonale, afin que leurs nombreuses faces évitent toute rotation des barres sur leur axe. Dans un but identique, les corps sphériques des rotules comporteront des facettes, comme on le verra plus loin dans la description détaillée des différents composants. En variante, on pourrait utiliser des barres de section circulaire et des rotules lisses, dont les surfaces extérieures seraient couvertes d'un revêtement anti-dérapant.

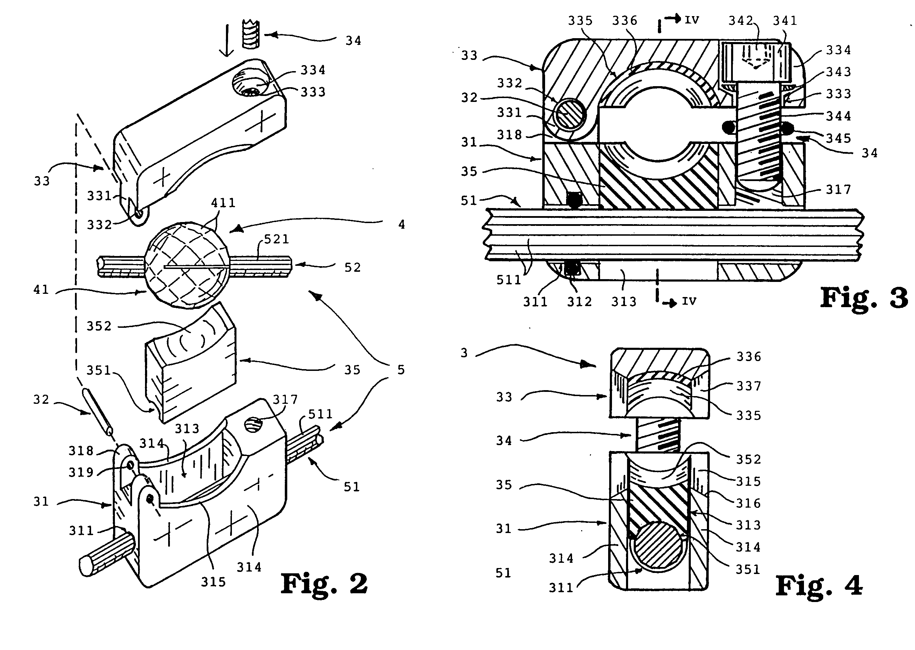

Le joint universel 3 représenté en détail aux figures 2 à 4 est constitué d'un corps 31 recevant un axe 32 sur lequel est articulée une mâchoire supérieure 33 destinée à maintenir la rotule 41 lorsque la vis 34 est serrée. Pour la clarté du dessin, la rotule 41 et la barre de liaison qui la traverse ne sont pas représentées aux figures 3 et 4.

Le corps 31 est de forme générale parallélipipédique. Il comporte un passage longitudinal 311 de section circulaire, dans lequel on peut introduire une barre de liaison 51. Comme visible dans la coupe de la figure 3, un joint O-ring 312 est disposé dans une gorge prévue à cet effet; il est destiné à retenir la barre 51 tout en autorisant son libre positionnement par rapport au joint universel au cours de la réduction de la fracture.

Le corps 31 présente de plus un dégagement interne 313 destiné à recevoir un élément de blocage 35 maintenu entre des ailes latérales 314. Vers le haut les ailes 314 présentent un dégagement arrondi 315 (figure 2) terminé par un biseau 316 (figure 4). Dans sa partie supérieure, le corps 31 possède une ouverture taraudée 317, perpendiculaire au passage 311, destinée à coopérer avec la vis 34, ainsi que deux oreilles 318 munies de passages 319 pour le maintien de l'axe 32.

La mâchoire supérieure 33 est aussi de forme générale parallélipipédique. Dans sa partie inférieure elle comporte à une extrémité une projection 331 munie d'un passage transversal 332 pour l'axe 32, et à l'autre extrémité une ouverture 333 dans le prolongement de l'ouverture taraudée 317 recevant la vis de serrage 34 dont la tête 341 est noyée dans un dégagement 334. La partie inférieure de la mâchoire 33 comporte encore un creusure de courbure sphérique 335 destinée à recevoir la rotule 41. Un revêtement 336 est appliqué dans cette creusure 335 pour favoriser la fixation de la rotule : le revêtement est constitué par une couche souple élastique ou est réalisé par traitement de surface. On notera à la figure 4 les dégagements arrondis biseautés 337 destinés à laisser libre passage pour une barre de liaison 52 traversant la rotule 41, tout comme les arrondis 315 du corps 31.

Les moyens de serrage du joint universel sont constitués par la vis 34 dont la tête 341 est munie d'une ouverture 342 destinée à recevoir un outil de serrage, par exemple à 6 pans. La tête 341 appuye sur une rondelle 343 destinée à coopérer avec le fond du dégagement 334 et sa partie filetée 344 vient en prise dans le taraudage 317 du corps 31. En outre on peut disposer un O-ring 345 sur le filetage 344 entre le corps 31 et la mâchoire 33, de manière à retenir la vis 34 lorsqu'elle n'est pas engagée dans le taraudage 317.

L'élément de blocage 35 est destiné à coopérer d'une part avec la rotule 41 et d'autre part avec la barre de liaison 51. A cet effet il présente sur toute sa longueur un dégagement semi-cylindrique 351 dans sa partie inférieure et une creusure 352 de forme sphérique dans sa partie supérieure. Le dégagement 351 est dimensionné pour correspondre à la section de la barre 51 et la creusure 352 correspond à la courbure de la rotule 41. Grâce à cet élément de blocage 35 agissant à la fois sur la rotule 41 et la barre de liaison 51, il est possible de positionner ces pièces et de les bloquer en une seule opération en agissant sur les moyens de serrage 34.

Pour augmenter l'accrochage entre ces pièces, on notera que la barre 51 est constituée par un cylindre à 12 pans 511 et que la rotule présente une succession de facettes 411, de telle manière que leurs arêtes puissent s'incruster dans l'élément de blocage 35, réalisé dans une matière moins dure que celles de la rotule et de la barre de liaison. En variante, on peut remplacer ces éléments à arêtes par un revêtement anti-dérapant formé par exemple de micro-billes disposées sur une barre de section circulaire ou une rotule parfaitement sphérique.

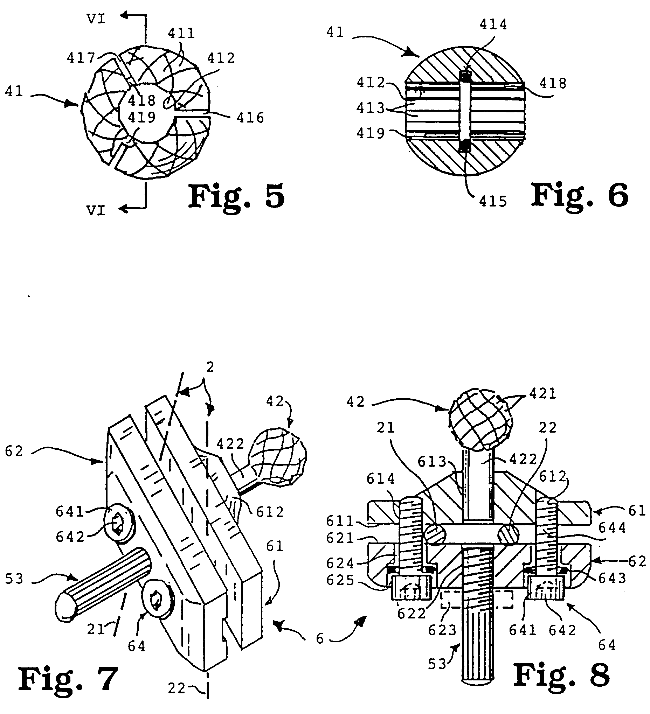

Dans la variante représentée à la figure 2, la rotule 41 est traversée par une barre de liaison 52. En se référant aux vues détaillées de la rotule des figures 5 et 6, on notera que la rotule 41 présente un passage central 412 destiné à recevoir avec jeu une barre de liaison de section dodécagonale. A cet effet, le passage central 412 comporte des cannelures 413 correspondant aux faces 521 de la barre de liaison 52. On notera dans la coupe de la figure 6 une gorge 414 recevant un O-ring 415 destiné à maintenir la barre 52 tant que la rotule 41 n'est pas serrée dans le joint universel de la figure 2.

La rotule 41 est munie de trois fentes radiales 416 et 417, régulièrement espacées sur sa périphérie et destinées à donner de l'élasticité à la rotule. La fente 416 est réalisée sur toute la longueur de la rotule tandis que les fentes 417 s'étendent sur une partie de sa longueur, des ponts 418 et 419 maintenant les segments constituant la rotule. En variante, on peut envisager un nombre pair de fentes 417, disposées en alternance d'un côté ou de l'autre sur la périphérie de la rotule.

Le joint universel des figures 2 à 4 peut également recevoir une rotule directement réalisée en bout d'une barre de liaison, comme représenté schématiquement à la figure 1. Elle peut selon les besoins maintenir aussi une rotule fixée à un autre constituant du fixateur externe, tel l'étau 6 d'orientation et de serrage des fiches osseuses, détaillé aux figures 7 et 8.

L'étau 6 est constitué de deux mâchoires 61 et 62 de forme générale parallélipipédique destinées à serrer les fiches osseuses 21 et 22. Il est à noter que les faces 611 et 621 des mâchoires 61 et 62 se faisant face sont planes, de manière à autoriser le maintien de fiches 21 et 22 disposées non parallèlement.

La mâchoire 61 comporte sur sa face extérieure un bossage 612 présentant un passage 613 destiné à la fixation d'une rotule 42. Dans cette variante, la rotule 42 présentant des facettes 421 d'accrochage est réalisée en bout d'une barre 422 fixée dans le passage central 613, par collage, soudure ou autre. En variante et pour permettre de modifier la longueur de la barre 422, on peut prévoir une fixation amovible de la rotule 42 dans la mâchoire 61. Celle-ci présente en outre deux ouvertures taraudées 614 destinées à recevoir les moyens de serrage de l'étau.

La mâchoire 62 comporte une ouverture 622 dans laquelle est fixée une barre de liaison 53. La longueur de celle-ci sera choisie en fonction des besoins et en outre, comme on le verra par la suite, il peut être intéressant de la supprimer en cours de traitement. La barre 53 est donc de préférence vissée et assurée par un contre-écrou 623, représenté en traitillés à la figure 8. La mâchoire 62 présente aussi deux passages 624 avec dégagements extérieurs 625 pour les moyens de serrage 64 de l'étau. L'écartement entre les passages 624 est donc le même que celui entre les ouvertures taraudées 614.

Les moyens de serrage 64 sont constitués par deux vis dont la tête 641 est munie d'une ouverture 642 destinée à recevoir un outil de serrage, par exemple à 6 pans. Chaque tête 641 appuye sur une rondelle 643 destinée à coopérer avec le fond du dégagement 625 et sa partie filetée 644 vient en prise dans le taraudage 614 de la mâchoire 61.

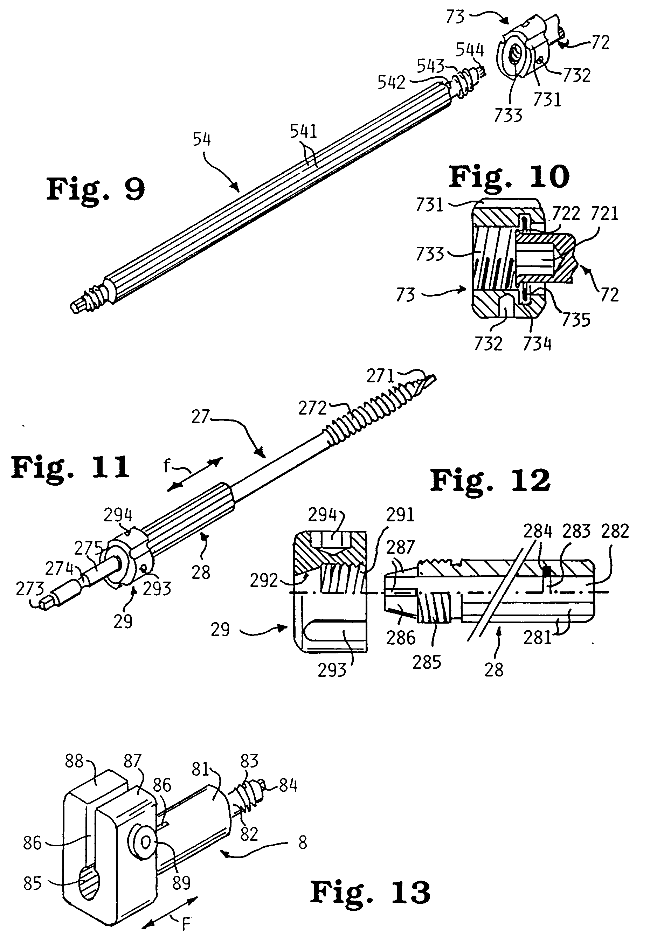

On a représenté à la figure 8 une barre 53 dont l'extrémité libre est arrondie. En variante on peut prévoir d'insérer dans l'étau une barre munie de moyens de fixation en bout, tels que ceux qui seront décrits en regard de la barre 54 de la figure 9.

Comme les barres de liaison décrites précédemment, cette barre 54 est de section dodécagonale et présente des pans 541 destinés à coopérer par exemple avec l'élément de blocage 35 des figures 2 à 4 ou avec les cannelures 413 pratiquée dans la rotule 41 des figures 5 et 6. Pour permettre sa liaison en bout avec les éléments de fixateurs décrits ici, l'une au moins de ses extrémités comporte un rétrécissement 542 muni d'un filetage 543, terminé par un prolongement de section carrée ou hexagonale 544 destiné à coopérer avec une ouverture correspondante d'une pièce 72, pour éviter toute rotation de la barre de liaison sur son axe.

De préférence, on utilisera des barres de liaison de différentes longueurs standards, par exemple les multiples de 5 cm, pour adapter le fixateur à chaque utilisation spécifique.

L'articulation 7 représentée dans la vue générale de la figure 1 est destinée à assurer l'orientation relative des deux barres 503 et 504 qui sont rendues solidaires de l'articulation par une pièce de liaison cylindrique 71 ou 72 et fixées au moyen d'écrous 73. Dans la variante représentée aux figures 9 et 10, la pièce cylindrique 72 présente à son extrémité libre un dégagement central 721, de forme correspondant au prolongement de section carrée ou hexagonale 544 de la barre de liaison 54, et est terminée par une collerette extérieure 722 destinée à maintenir l'écrou 73.

L'écrou 73 présente extérieurement des cannelures 731 entre lesquelles sont prévues des ouvertures 732 destinées à recevoir un outil de serrage, par exemple à 6 pans. L'écrou 73 présente intérieurement un taraudage 733 correspondant au filetage 543 de la barre de liaison et une gorge 734 apte à recevoir un clips 735 dimensionné de telle manière qu'il soit maintenu par la collerette 722. En variante, le clips 735 peut être remplacé par une série de billes, introduites par un passage pratiqué radialement dans l'écrou 73.

Les fiches insérées dans les os utilisées dans le fixateur selon l'invention peuvent être des fiches auto-taraudeuses comme la fiche 27 détaillée à la figure 11. Chaque fiche comporte une extrémité de taraudage 271, suivie d'un filetage 272 destiné à la maintenir dans le fragment osseux. Son extrémité opposée se termine par un carré 273 destiné à coopérer avec un outil permettant d'effectuer les rotations nécessaires pour insérer la fiche dans l'os. Un dégagement 274 est prévu sur la partie lisse 275 de la fiche pour permettre son maintien dans un mandrin.

Dans la variante de la figure 11, une douille intermédiaire 28 est prévue sur la partie lisse 275 de la fiche de manière à porter le diamètre de celle-ci à celui des barres de liaison 51, 52, etc, pour que les composants du fixateur soient modulaires. La douille 28 est constituée par un tube présentant extérieurement 12 pans 281 correspondant aux pans 511, 521, 531 ou 541 des barres déjà décrites. Intérieurement la douille comporte un passage central 282 de section circulaire destiné à recevoir la partie lisse 275 de la fiche. Le passage 282 présente une gorge 283 recevant un O-ring 284 destiné à retenir la fiche 27 en place tant que la douille n'est pas serrée. L'une des extrémités de la douille comporte un filetage 285 terminé par une partie tronconique 286 présentant des fentes radiales 287 régulièrement espacées sur sa périphérie et conférant une certaine élasticité à l'extrémité de la douille. Le filetage 285 est destiné à coopérer avec l'écrou 29 et plus particulièrement avec son taraudage 291. Le fond de l'écrou présente un dégagement tronconique 292 destiné à coopérer avec la partie tronconique 286 de la douille pour resserrer celle-ci sur la fiche 27. L'écrou 29 présente extérieurement des cannelures 293 permettant de le serrer à la main ainsi que des ouvertures 294 destinées à recevoir un outil de serrage utilisé lors du blocage de la douille le long de la partie lisse 275 de la fiche. On notera que de préférence on utilisera le même outil de serrage dans ces ouvertures 294 que dans les ouvertures 732 de l'écrou 73 de fixation d'une barre en bout de l'articulation des figures 9 et 10.

Le raccord télescopique 8 de la figure 13 permet de mettre bout à bout deux barres de liaison, à une distance qui peut être réglée dans le sens de la flèche F pour que les barres formant le fixateur ne s'étendent pas au-delà des raccords dans lesquels elles sont fixées. Il présente une partie tubulaire 81 dont l'extrémité comporte un rétrécissement 82 muni d'un filetage 83, terminé par un prolongement de section carrée ou hexagonale 84 destiné à coopérer avec une ouverture correspondante de la pièce adjacente, pour éviter toute rotation du raccord 8 sur son axe. Les dimensions du filetage 83 et de son prolongement 84 sont les mêmes que celles du filetage 543 et du prolongement 544 de la fiche 54, pour autoriser la liaison à d'autres constituants du fixateur externe selon l'invention, au moyen d'écrous semblables à l'écrou 73 des figures 9 et 10.

La partie tubulaire 81 comporte à son autre extrémité une ouverture centrale 85 de section dodécagonale, destinée à recevoir une barre de liaison qui dans ce cas particulier peut se terminer par une partie arrondie, comme la barre 53 de la figure 8.

Une fente d'élasticité 86 est prévue sur la partie tubulaire 81, et est bordée extérieurement par deux prolongements 87 et 88 destinés à recevoir des moyens de serrage 89 constitués par une vis passant librement dans un passage pratiqué dans le prolongement 87 et venant en prise dans un taraudage pratiqué dans le prolongement 88. Il est à noter que la tête de vis présente la même ouverture à 6 pans que tous les éléments destinés à être serrés décrits précédemment.

Dans une variante non représentée au dessin, on peut prévoir de disposer à chaque extrémité de la partie tubulaire 81 une ouverture centrale 85 destinée à recevoir une barre et des prolongements 87 et 88 aptes à être serrés l'un vers l'autre par des moyens de serrage 89.

Il est évident que tous les composants décrits jusqu'ici ne présentent aucune arrête vive, pour éviter de blesser le chirurgien ou le patient. La majorité des pièces seront réalisées en alliage léger ou en matériau composite, pour rendre l'ensemble aussi léger que possible d'une part et pour qu'il soit transparent aux rayons X d'autre part.

Au cours de l'opération, le praticien commencera par mettre en place les fiches insérées dans les fragments osseux, en tenant compte uniquement de critères anatomiques.

En se référant à la figure 1, les fiches seront alors serrées soit dans les étaux 65 et 66 (décrits en détail en regard des figures 7 et 8), soit dans un joint universel 38, et plus précisément dans son passage longitudinal après avoir été introduites dans une douille intermédiaire 28 (détaillée par rapport aux figures 11 et 12) et fixées au moyen de l'écrou de serrage 29.

Selon la configuration spécifique, on choisira alors le type de rotules 46 à 48 à utiliser dans le joint universel, avant de mettre en place les barres de liaison 503 et 504. Sous rayons X, la fracture sera réduite de manière à ce que les fragments osseux retrouvent leur position normale. Afin de rigidifier l'ensemble de manière optimale, on peut encore renforcer le cadre du fixateur par les barres 501, 502 et 506, 507, même 505 si nécessaire, ces barres étant fixées comme représenté à la figure 1 par exemple, ou de toute autre manière équivalente.

Comme on l'a déjà mentionné, on peut enlever l'une des barres après le début de la consolidation osseuse, afin de diminuer l'encombrement et le poids de l'ensemble tout en autorisant un léger mouvement des fragments osseux au niveau de la fracture, mouvemement ayant tendance à favoriser la formation de cal.

Grâce à la construction modulaire de tous les composants décrits ici, il est à noter qu'un outil de serrage unique est utilisé pour le serrage des vis 34, 64, 74, 89 et 96 utilisées dans le joint universel 3, l'étau 6, l'articulation 7, les raccords télescopique 8 et orientable 9 respectivement.

Ce même outil peut être introduit dans les ouvertures telles que celles détaillées sous référence 732 dans la coupe de l'écrou 73 de la figure 10. De même, l'écrou 29 de serrage de la fiche 25 et les divers écrous schématisés à la figure 1 pour la fixation des barres dans les raccords 7 et 8.

## Revendications

1. Fixateur externe comportant :
- des fiches (2, 21 à 27) insérées dans les fragments osseux,
- une ou plusieurs barres de positionnement constituant le cadre extérieur du fixateur,
- un ou plusieurs raccords d'articulation (7, 8, 9) entre les barres et les fiches ou les barres,
les fiches (2, 21 à 27) étant au moins indirectement solidaires d'un joint universel (3; 36 à 38) à rotule amovible (4; 41, 42, 46 à 48) apte à assurer le positionnement indépendant des fiches par rapport à l'une desdites barres (5; 51 à 57; 501 à 507),
caractérisé en ce que le joint universel est composé de :
- un corps partiellement sphérique constituant une rotule (4; 41, 42, 46 à 48),
- une première partie (33) munie d'une première creusure (335) de forme générale sphérique,
- une seconde partie (31) munie d'un passage longitudinal (311) et d'un dégagement (313) faisant face à la première creusure,
- un élément de blocage (35) muni d'une seconde creusure (352) de forme générale sphérique et disposé dans ledit dégagement,
- au moins une vis de serrage (34) disposée sensiblement perpendiculairement audit passage longitudinal et apte à bloquer à la fois la rotule et une pièce cylindrique de la barre ou de la fiche introduite dans le passage longitudinal.

2. Fixateur externe selon la revendication 1, caractérisé en ce que les première et seconde parties du joint universel sont articulées sur un axe (32) disposé sur les faces opposées à la vis de serrage (34).

3. Fixateur externe selon la revendication 1, caractérisé en ce que les première et seconde parties du joint universel présentent des dégagements latéraux (337, 315).

4. Fixateur externe selon la revendication 1, caractérisé en ce que l'élément de blocage présente, sur sa face opposée à la seconde creusure, un dégagement longitudinal (351) apte à coopérer avec la pièce cylindrique introduite dans le passage (311).

5. Fixateur externe selon la revendication 4, caractérisé en ce que ladite pièce cylindrique est constituée par une barre de positionnement (5; 51 à 56; 501 à 507) ou par une fiche entourée d'une douille (28) munie de fentes longitudinales (287) et serrée sur la fiche au moyen d'un écrou (29).

6. Fixateur externe selon la revendication 1, caractérisé en ce que la surface extérieure de la pièce cylindrique et/ou de la rotule est munie d'un revêtement antidérapant.

7. Fixateur externe selon la revendication 1, caractérisé en ce que la surface extérieure de la pièce cylindrique et/ou de la rotule est constituée par des facettes de blocage.

8. Fixateur externe selon la revendication 1, caractérisé en ce que la rotule (4;41,47,48) comporte un passage central (412) pour une barre de liaison et des fentes d'élasticité (416,417).

9. Fixateur externe selon la revendication 8, caractérisé en ce que la rotule est munie de moyens de liaison, fixes ou amovibles, à un étau de serrage des fiches (6; 65,66).

10. Fixateur externe selon la revendication 9, caractérisé en ce que la ou les mâchoires (61, 62) de l'étau comportent sur leur face opposée aux fiches des moyens de fixation d'une barre (53) ou d'un corps cylindrique dont une extrémité est munie d'une rotule fixe (42,46) ou amovible (47,48).

11. Fixateur selon la revendication 1 comportant en outre un raccord télescopique (8) disposé en bout de deux barres (506, 507), caractérisé en ce que ledit raccord comporte une partie tubulaire (81) dont au moins une extrémité présente une ouverture centrale (85) destinée à recevoir une barre et des prolongements (87,88) aptes à être rapprochés par des moyens de serrage (89).

12. Fixateur selon les revendications 1 à 11, caractérisé en ce que les têtes des vis de serrage (34, 64, 74, 89) comportent toutes le même dégagement apte à recevoir un outil de serrage.

13. Fixateur selon la revendication 5, caractérisé en ce que l'écrou de serrage (29, 73, 623) comporte un dégagement apte à recevoir un outil de serrage.

## Claims

1. External fixator comprising:
- pins (2, 21 to 27) inserted into the bone fragments,
- one or more positioning bars constituting the outer frame of the fixator,
- one or more connecting pieces (7,8,9) for articulation between the bars and the pins or the bars,
the pins (2, 21 to 27) being at least indirectly integral with a universal joint (3, 36 to 38) with a removable ball (4; 41, 42, 46 to 48) capable of ensuring the independent positioning of the pins relative to one of the said bars (5; 51 to 57; 501 to 507), characterized in that the universal joint consists of:
- a partially spherical body constituting a ball (4; 41, 42, 46 to 48),
- a first part (33) provided with a first hollow (335) of general spherical shape,
- a second part (31) provided with a longitudinal passage (311) and a clearance (313) facing the first hollow,
- a blocking element (35) provided with a second hollow (352) of general spherical shape and arranged in the said clearance,
- at least one locking screw (34) arranged substantially perpendicular to the said longitudinal passage and able to block at one and the same time the ball and a bar or a pin cylindrical piece introduced into the longitudinal passage.

2. Fixator according to Claim 1, characterized in that the first and second parts of the universal joint are articulated on a shaft (32) arranged on the faces opposite the locking screw (34).

3. Fixator according to Claim 1, characterized in that the first and second parts of the universal joint have lateral clearances (337, 315).

4. Fixator according to Claim 1, characterized in that the blocking element has, on its face opposite the second hollow, a longitudinal clearance (351) able to cooperate with the cylindrical piece introduced into the passage (311).

5. Fixator according to Claim 4, characterized in that the said cylindrical piece consists of a positioning bar (5; 51 to 56; 501 to 507) or of a pin surrounded by a chuck (28) provided with longitudinal slots (287) and closed on the pin by means of a nut (29).

6. Fixator according to Claim 1, characterized in that the outer surface of the cylindrical piece and/or the ball is provided with an anti-slip coating.

7. Fixator according to Claim 1, characterized in that the outer surface of the cylindrical piece and/or the ball consists of blocking facets.

8. Fixator according to Claim 1, characterized in that the ball (4; 41, 47, 48) comprises a central passage (412) for a connecting bar and elasticity slots (416,417).

9. Fixator according to Claim 8, characterized in that the ball is provided with fixed or removable means of connection to a vice (6; 65, 66) for clamping the pins.

10. Fixator according to Claim 9, characterized in that the jaw or jaws (61, 62) of the vice comprise, on their face opposite the pins, means for fixation of a bar (53) or of a cylindrical body, of which one end is provided with a ball which is fixed (42, 46) or removable (47, 48).

11. Fixator according to Claim 1, comprising additionally a telescopic connecting piece (8) arranged at the end of two bars (506, 507), characterized in that the said connecting piece comprises a tubular part (81) of which at least one end has a central opening (85) intended to receive a bar, and extensions (87,88) capable of being brought together by means of clamping means (89).

12. Fixator according to Claims 1 to 11, characterized in that the heads of the locking screws (34, 64, 74, 89) all comprise the same clearance for receiving a clamping tool.

13. Fixator according to Claim 5, characterized in that the clamping nut (29, 73, 623) comprises a clearance capable of receiving a clamping tool.

## Patentansprüche

1. Äußere Fixatur zur Osteosynthese, welche folgendes aufweist:
- Steckverbinder (2, 21 bis 27), welche in Knochenteile eingeschoben werden,
- eine oder mehrere Positionierstangen, welche den äußeren Rahmen der Fixatur bilden,
- eine oder mehrere Gelenkverbindungen (7, 8, 9), welche zwischen den Stangen und den Steckverbindern oder aber zwischen den Stangen angeordnet sind,
wobei die Steckverbinder (2, 21 bis 27) zumindest indirekt mit einem Kardangelenk (3; 36 bis 38) mit löslichem Kugelgelenk (4; 41, 42, 46 bis 48) verbunden sind, welches geeignet ist, eine unabhängige Positionierung der Steckverbinder gegenüber einer der Stangen (5; 51 bis 57; 501 bis 507) zu ermöglichen,
**dadurch gekennzeichnet**, **daß**
das Kardangelenk aus folgendem besteht:
- einem teilweise kugelförmigen Körper, welcher ein Kugelgelenk (4; 41, 42, 46 bis 48) bildet,
- einem ersten Teil (33) mit einer ersten weitgehend kugelförmigen Einsenkung (335),
- einem zweiten Teil (31) mit einem längsförmigen Durchgang (311) und einem der ersten Einsenkung gegenüberliegenden Freistich (313),
- einem Verriegelungselement (35) mit einer zweiten weitgehend kugelförmigen Einsenkung (352), welches in diesen Freistich eingesetzt ist,
- mindestens einer Feststellschraube (34), welche weitgehend rechtwinklig zu dem längsförmigen Durchgang angeordnet und geeignet ist, sowohl das Kugelgelenk als auch einen zylindrischen Abschnitt der in den längsförmigen Durchgang eingeschobenen Stange oder des Steckverbinders zu blockieren.

2. Äußere Fixatur nach Anspruch 1,
**dadurch gekennzeichnet**, **daß**
die ersten und zweiten Abschnitte des Kardangelenkes an einer Welle (32) angelenkt sind, welche an den der Feststellschraube (34) gegenüberliegenden Seiten angeordnet ist.

3. Äußere Fixatur nach Anspruch 1,
**dadurch gekennzeichnet**, **daß**
die ersten und zweiten Abschnitte des Kardangelenkes seitliche Freistiche (337, 315) aufweisen.

4. Äußere Fixatur nach Anspruch 1,
**dadurch gekennzeichnet**, **daß**
das Verriegelungselement an seiner der zweiten Einsenkung gegenüberliegenden Seite einen längsförmigen Freistich (351) aufweist, welcher mit dem in den Durchgang (311) eingeschobenen zylindrischen Teil zusammenwirkt.

5. Äußere Fixatur nach Anspruch 4,
**dadurch gekennzeichnet**, **daß**
dieser zylindrische Teil aus einer Positionierstange (5; 51 bis 56; 501 bis 507) oder aus einem Steckverbinder besteht, welcher von einer mit Längsschlitzen (287) versehenen Hülse (28) umgeben ist, die mit Hilfe einer Mutter (29) an dem Steckverbinder festgezogen wird.

6. Äußere Fixatur nach Anspruch 1,
**dadurch gekennzeichnet**, **daß**
die Außenfläche des zylindrischen Teils und/oder des Kugelgelenkes mit einem rutschfesten Überzug versehen ist.

7. Äußere Fixatur nach Anspruch 1,
**dadurch gekennzeichnet**, **daß**
die Außenfläche des zylindrischen Teils und/oder des Kniegelenkes aus Verriegelungsfasen besteht.

8. Äußere Fixatur nach Anspruch 1,
**dadurch gekennzeichnet**, **daß**
das Kugelgelenk (4; 41, 47, 48) einen zentralen Durchgang (412) für eine Verbindungsstange sowie Federschlitze (416, 417) aufweist.

9. Äußere Fixatur nach Anspruch 8,
**dadurch gekennzeichnet**, **daß**
das Kugelgelenk mit festen oder löslichen Mitteln für eine Verbindung mit einer Federspannschraube (6; 65, 66) für die Steckverbinder angeschlossen ist.

10. Äußere Fixatur nach Anspruch 9,
**dadurch gekennzeichnet**, **daß**
die Backen (61, 62) der Federspannschraube an ihrer den Steckverbindern gegenüberliegenden Fläche Mittel für die Befestigung einer Stange (53) oder eines zylindrischen Körpers aufweisen, von denen ein Endteil mit einem festen (42, 46) oder löslichen (47, 48) Kugelgelenk ausgestattet ist.

11. Äußere Fixatur nach Anspruch 1, welche außerdem eine Teleskopverbindung (8) aufweist, die an den Enden von zwei Stangen (506, 507) angeordnet ist,
**dadurch gekennzeichnet**, **daß**
diese Verbindung einen rohrförmigen Teil (81) aufweist, von dem mindestens ein Endteil mit einer zentralen Öffnung (85) versehen ist, welche eine Stange sowie Verlängerungen (87, 88) aufnehmen kann, welche mit Hilfe von Festellmitteln (89) aneinander angenähert werden können.

12. Äußere Fixatur nach einem der Ansprüche 1 bis 11,
**dadurch gekennzeichnet**, **daß**
die Köpfe der Feststellschrauben (34, 64, 74, 89) jeweils einen gleichartigen Freistich aufweisen, in den ein Schraubwerkzeug eingesetzt werden kann.

13. Äußere Fixatur nach Anspruch 5,
**dadurch gekennzeichnet**, **daß**
die Feststellmuttern (29, 73, 623) jeweils einen Freistich aufweisen, in den ein Schraubwerkzeug eingesetzt werden kann.
